# EUROPEAN PATENT APPLICATION

(11) **EP 1 600 131 A1**
(43) Date of publication of application: **30.11.2005**
(21) Application number: 04012225.1
(22) Date of filing: 24.05.2004
(51) Int. Cl.: A61F 13/15, B65D 75/04

(54) **Individually packaged cleaning wipe**

(71) Applicant: THE PROCTER & GAMBLE COMPANY, Cincinnati, Ohio 45202 (US)
(72) Inventor: Nijs, Brigitte Luisette Camille, 65779 Kelkheim (DE); Frank, Martin Werner, 61118 Bad Vilbel (DE)
(74) Representative: Kremer, Véronique Marie Joséphine

(57) **Abstract**

The present invention relates to the field of sanitary absorbent articles of personal hygiene. A particular focus of the present invention are absorbent articles of feminine hygiene. The present invention provides individually-packaged absorbent articles in combination with individually-packaged wipes. At least one of the package of the absorbent article and the package of the wipe is provided with an attachment means on its outside, which allows attachment of the other package to it. Thereby the user of the absorbent article is given the opportunity to combine an individually-packaged absorbent article with an individually-packaged wipe on demand.

## Description

### Cross Reference to Related Applications

### Field of Invention

The present invention relates to the field of sanitary absorbent articles of personal hygiene. A particular focus of the present invention are absorbent articles of feminine hygiene. The present invention provides individually-packaged absorbent articles in combination with individually-packaged wipes. At least one of the package of the absorbent article and the package of the wipe is provided with an attachment means on its outside, which allows attachment of the other package to it. Thereby the user of the absorbent article is given the opportunity to combine an individually-packaged absorbent article with an individually-packaged wipe on demand.

### Background of the Invention

Absorbent articles for absorbing and handling body exudates, such as diapers, sanitary napkins, panty liners, bed pads and the like are widely known in the art. Due to the different hygienic needs articles like sanitary napkins are mostly individually wrapped, whereas diapers, panty liners or bed pads are packaged as a stack in a common package. It has also been recognized that changing such absorbent articles can be a hygienic challenge, as portions of the user's body need to be cleaned as well as the hands of the person changing the article may become contaminated. This is perceived as embarrassing and also handling of the soiled article and a new absorbent article for replacement is seriously affected by this.

It is widely known in the art to use wipes for cleaning of skin portions, which were soiled with body exudates. For instance the use of moistened baby wipes is widely practiced. Such wipes are made of a textile material and are oftentimes provided with a moist lotion, which eases cleaning and delivery skin benefits to the skin portion treated therewith. For maintaining the moistness of the wipe these wipes are packaged in wrappers with barrier properties. Exemplary packaged moistened wipes are disclosed e.g. in US 4,428,477. Typically, cleaning wipes are packaged in stacks in a common package, such as in case of moist baby wipes. In other instances, such as in case of cleaning wipes supplied in airplanes, there is only one wipe individually packaged in a wrapper.

Currently, when attempting to change a soiled absorbent article, such as a loaded sanitary napkin, the user or the person changing the article has to carry the package containing the article for changing as well as another package containing the wipe for cleaning. This is clearly disadvantageous because opening / handling and disposing of a multiplicity of items and especially packages is required. Solutions to this problem are suggested by e.g. US 5,350,067; US 4,808,175 or US 5,569,230. These documents suggest individual absorbent articles comprising an individually-packaged moistened wipe in or on the package of the absorbent article. In other words, absorbent article and wet wipe are always fixedly combined with each other. These combined absorbent articles are an improvement over prior approaches because the number of parts and also waste parts, which must be handled, is reduced as the packages of the absorbent article and the wipe are connected with each other. The disadvantage of such configurations is that a high complexity and precision in terms of manufacturing is required in order to achieve the combination of the individually-packaged absorbent article and the individually-packaged wipe at the typical speeds of production lines. As a result, manufacturing of such combined articles as suggested by the prior art is rather expensive. Further, such combined products are needed by the consumer only in special situations and not all the time. For example, a woman typically needs a cleaning wipe upon changing a sanitary napkin when she is out of home, and often wants to use it during mainly the high flow days. In other instances women might not feel the same need to have a cleaning wipe with her sanitary napkin. Therefore, having to buy all the time or full packages with such rather expensive combined articles would be conceived in many instances as either unnecessary, or alternatively would create a storage issue in the woman's bathroom, as she had to store normal as well as combined articles and thus consume more storage space.

Consequently, it would be desirable to provide absorbent articles having the advantages of combined articles as disclosed in the prior art in terms of increased convenience by reducing the number of items to be handled during the change, but which are however more flexible in terms of usage and which could be provided to the consumer at lower cost.

### Summary of the Invention

The above-described need has been addressed by the present invention by providing a kit comprising at least one, preferably a multiplicity of individually-packaged absorbent articles, packaged together with at least one, preferably a multiplicity of individually-packaged cleaning wipes. The package of either the absorbent article or preferably the cleaning wipe are provided with an attachment means, preferably an adhesive on their outside, which allows attachment of the individually-packaged cleaning wipe to the individually-packaged article.

The kit of the present invention provides the user with the possibility to combine absorbent article and wipe by attaching a packaged wipe to the package of the absorbent article. By this, a combined absorbent article is formed only then the user feels a need therefore. Further, since the individually-packaged absorbent article and the individually-packaged cleaning wipe are provided to the consumer separately in a common package, a significant decrease of complexity and thus a significant cost reduction can be achieved upon producing such combined absorbent articles.

The present invention also encompasses the individually-packaged cleaning wipe alone, having the attachment means, preferably an adhesive for attachment to the package of the absorbent article on its package outside.

The invention further encompasses an individually-packaged cleaning wipe, which is packaged in a package made of a laminate material comprising a layer of polyethylene (PE hereinafter) as the outermost bottom layer and a copolymer layer of polypropylene (PP) and PE as the outermost top layer, wherein the PE side of said copolymer layer is facing outwardly.

All documents cited are, in relevant part, incorporated herein by reference; the citation of any document is not to be construed as an admission that it is prior art with respect to the present invention.

### Brief Description of the Drawings

### Detailed Description of the Invention

The term 'absorbent article' is used herein in a very broad sense including any article being able to receive and/or absorb and/or contain and/or retain fluids and/or exudates, especially bodily fluids/bodily exudates. The absorbent article, which is referred to in the present invention typically comprises a fluid pervious topsheet as the wearer-facing layer, a fluid impervious backsheet as the garment-facing layer that is preferably water vapour and/or gas pervious and an absorbent core comprised there between. Furthermore, absorbent articles in the context of the present invention are provided with a means for their attachment to the user's garment, in particular with an adhesive. Preferred absorbent articles in the context of the present invention are disposable absorbent articles. Typical disposable absorbent articles according to the present invention are absorbent articles for personal hygiene, such as baby care articles like baby diapers; incontinence pads and perspiration pads like underarm sweat pads or hat bands. Particularly preferred disposable absorbent articles are absorbent articles for feminine hygiene like sanitary napkins and panty liners.

By 'body fluid' or 'body exudates' it is meant herein any fluid produced by the human body including for instance perspiration, urine, blood, menstrual fluids, vaginal secretions, bowel movement and the like.

The term 'disposable' is used herein to describe articles, which are not intended to be laundered or otherwise restored or reused as an article (i.e. they are intended to be discarded after a single use and preferably to be recycled, composted or otherwise disposed of in an environmentally compatible manner).

The term 'use', as used herein, refers to the period of time that starts when the absorbent article is actually put in contact with the anatomy of the user.

'Individually packaged' as used herein comprises that only one item (absorbent article or moist wipe) is packaged in an individual package as well as a small number of items, i.e. up to five, is packaged in one package.

'Combined absorbent article' as used herein means an individually-packaged absorbent article with an individually-packaged cleaning wipe attached thereto. The manner of attachment can be either fixedly or releasably.

The present invention provides a kit comprising at least one individually-packaged absorbent article and at least one individually-packaged wipe. In a preferred embodiment herein, each package comprises only one absorbent article, or one cleaning wipe, respectively.

The attachment means on the outside of the package of the cleaning wipe is selected and applied such that a sufficient degree of adhesive attachment between the package of the absorbent article and the package of the cleaning wipe is generated. Adhesives are particularly preferred for this purpose. The adhesive is preferably a pressure-sensitive adhesive. Suitable examples therefore are hot melt, cold melt or solvent-based adhesives pressure-sensitive adhesives. Basically every adhesive known in the art for attaching materials like plastic films or nonwovens to each other is suitable for use herein. The adhesive herein can be present on the outer surface of the package of the cleaning wipe in a number of manners. Suitable herein are application as a continuously coated area as well as application as a pattern. Suitable patterns are dots, stripes, spirals, beads and the like. Typically the package of the cleaning wipe is substantially flat with two opposing major surfaces. According to the present invention it is preferred that the adhesive is only present on one surface of the package. The adhesive can cover the whole area of the surface or only part thereof. Instead of an adhesive a double-sided adhesive tape can be used herein. The adhesive herein is preferably inactive; for attachment to the package of the absorbent article it has to be activated by the user. Inactivation of the adhesive is possible by e.g. covering the adhesive by a release paper or by encapsulating the adhesive e.g. in microcapsules. Activation can therefore be facilitated by removing the release paper or by applying pressure or friction to the microcapsules in order to rupture them open. However, there are also embodiments according to the present invention where the adhesive does not need to be inactivated by special measures. This is for instance the case when a stack of packages containing individual cleaning wipes is used, where each wipe package is attached with its adhesive to another one, which then serves as a release item.

In another execution the adhesive is applied onto the outer surface of the package of the individually-packaged absorbent article.

In further embodiments herein the package of the absorbent article and the package of the wipe are combined by mechanical fasteners instead by an adhesive. In an exemplary embodiment the package of the absorbent article comprises a nonwoven as outer layer, whereas the package of the wipe comprises a so-called hook tape on its outside. The hooks of the hook tape can engage into the nonwoven when the package of the wipe is brought into contact with the package of the absorbent article, whereby attaching both packages to each other.

The package of the cleaning wipe herein preferably has barrier properties for preventing evaporation of moisture from the cleaning wipe. Suitable barrier materials are laminates, where a layer of EVOH (ethylene vinyl alcohol) or PVOH (propylene vinyl alcohol) or aluminium is sandwiched between polymeric film layers, such as LDPE (low density polyethylene). Further suitable barrier materials for forming the package for the cleaning wipe herein are metallised, particularly aluminised plastic films; metal foils; oriented polyethylene terephtalate (PET); PETG (glycol-modified PET); oriented polyamide; aromatic polyamide; or polymeric films like polyethylene films with special lacquer coatings, which provide the polymeric film with the vapour barrier functionality. Basically every material known in the art for this purpose is suitable for use herein. A very suitable laminate material for this purpose is for instance disclosed in EP 696,991. This material is a laminate with a PE (polyethylene) bottom layer, on top of which a layer of aluminium foil or optionally aluminized PE is attached with an adhesive. On top of the aluminium or aluminized layer a co-extruded layer of OPP (oriented polypropylene) and PE is attached with an adhesive, with the OPP side facing the aluminium or aluminized layer and the PE side facing outward. For forming a package this material is folded over and the facing PE surfaces are heat bonded to each other. Upon opening such a package the PE part of the co-extruded OPP/PE layer is pulled off the OPP part. Because of this, the package made from this material is only suitable for one single opening and is not reclosable. The package of the wipe can comprise opening aids for facilitating opening of the package. Exemplary opening aids are tear tapes, cuts or the like.

In a particularly preferred embodiment herein the cleaning wipe is moist. Moistening is most typically facilitated by applying a lotion to the wipe. Suitable lotions are aqueous or non-aqueous ones, having fluid-like viscoelastic properties at room temperature. Lotions for use herein are increasing the cleaning capabilities of the wipe by aiding removal of soiling from the skin.

Further, lotions for use herein preferably also provide skin benefits by reducing friction between the skin and the wipe and / or by containing materials like Aloe vera or chamomile, which increase skin health. Lotions are provided to the wipe in an amount such that the wipe is noticeably moist to the user and that the cleaning capacities of the wipe are increased. Applying lotion to the wipe in an amount such that the lotion drips off from the wipe without exertion of pressure, such as by wringing out, is less desirable herein. Examples for suitable lotions are disclosed in EP-A-808,151; EP-A-763,341 or WO 00/57843.

The individually-packaged cleaning wipe according to another aspect of the present invention is packaged in a package, which is made of a package material comprising an inner layer comprising polyethylene (PE), an outer layer comprising a copolymer of polyethylene (PE) and polypropylene (PP) and a subjacent intermediate layer comprising polypropylene (PP).

By using a polymer-containing polyethylene in both the inner and the outer layer, in addition to obtaining a gas proof strong seal when welding inner face to inner face, i.e. the PE-containing layer is welded onto itself, it is possible when welding inner face to outer face to provide a gas-proof seal and to control the strength of the seal, i.e. the peel strength, very accurately. On the whole, a gas proof or vacuum proof packing is thus obtained, said packing being sealed by means of a peel seal, whose properties can be controlled very accurately. These advantageous properties have made it possible to manufacture the package in a rational manner on a vertical package form, fill and seal machine, which has not been possible previously due to the problems of controlling the quality of the welding.It is thus an essential feature of the package according to the invention that the side edge weldings, which are heavily stressed, when the product falls thereon during filling, are fusion seals having sufficient strength when hot (hot tack strength) to resist said stresses. This in constrast to known peel seal packages, wherein the side edge welds are peelable.

The cleaning wipe is packaged in a package made of a flexible packing material by folding a sheet of material having an inner face and an outer face. The package further comprises a pouch portion having a front wall and a back wall and a flap extending from the back wall and folded down onto the front wall about a folding line being essentially aligned with the upper free edge of the front wall. The package has the inner face sealed to the outer face in the overlapping portion between the flap and the front wall by means of a longitudinal welding seam. The inner face is welded to the outer face along two side edge portions, and the outer face is welded to the inner face in the adjacent areas of the overlapping portion for obtaining a closed package enclosing a packed product. The packing material provides a peel seal when the inner face is welded to the outer face, and a strong seal (fusion seal) when the inner face is welded to the inner face. Seen relative to the package the packing material comprises an inner layer comprising polyethylene (PE), an outer layer comprising a copolymer of polyethylene and polypropylene and a subjacent intermediate layer comprising polypropylene (PP).

According to the invention, the inner layer may be a copolymer of PE and PP differing from the copolymer in the outer layer.

The cleaning wipe o this aspect of the invention can be of use in various fields. Examples are cleaning wipes for personal hygiene, such as baby wipes, wipes for feminine hygiene or moist toilet paper, or cleaning wipes in the household field for e.g. surface cleaning.

While particular embodiments of the present invention have been illustrated and described, it would be obvious to those skilled in the art that various other changes and modifications can be made without departing from the spirit and scope of the invention. It is therefore intended to cover in the appended claims all such changes and modifications that are within the scope of this invention.

## Claims

1. An individually-packaged cleaning wipe, **characterized in that** the package of said wipe is provided with an attachment means on its outside for attachment to a surface.

2. The wipe of claim 1, wherein said attachment means is an adhesive.

3. The wipe of claim 2, wherein said adhesive is inactive and requires activation by the user.

4. The wipe of claim 3, wherein said adhesive is covered by a release paper and is activated by the removal of said release paper.

5. The wipe of claim 3, wherein the adhesive is encapsulated and is activated by breaking the encapsulation by exertion of pressure.

6. The wipe of claim 1, wherein said attachment means is a mechanical fastener of the hook-loop type.

7. The wipe of any of the preceding claims, wherein said package is substantially flat with two opposing surfaces and is provided with said adhesive or said mechanical fastener on one of said surfaces.

8. The wipe of any of the preceding claims, wherein said package is made of a material having barrier properties, preventing evaporation of moisture from said wipe.

9. The wipe of claim 8, said material of said package comprising a bottom layer of polyethylene, a top layer of co-extruded oriented polypropylene and polyethylene and an intermediate layer of aluminium foil or aluminized polyethylene being arranged between said bottom and said top layer, wherein said top layer is oriented such that said intermediate layer is faced by the oriented polypropylene side of said top layer.

10. The wipe of any of the preceding claims, wherein said package is provided with opening aids.

11. The wipe of any of the preceding claims, wherein said wipe is moistened.

12. The wipe of any of the preceding claims, wherein each package comprises only one cleaning wipe.

13. Use of a wipe according to any of the preceding claims for forming a combined absorbent article by attaching said package containing said wipe to the package of an absorbent article through said adhesive.

14. Kit comprising at least one individually-packaged absorbent article and at least one individually-packaged cleaning wipe according to any of claims 1-12.

15. The kit of claim 14, wherein each package comprises only one absorbent article or one cleaning wipe, respectively.

16. An individually-packaged cleaning wipe, wherein said cleaning wipe is packaged in a package made of a flexible packing material by folding a sheet of material having an inner face and an outer face and comprising a pouch portion having a front wall and a back wall and a flap extending from the back wall and folded down onto the front wall about a folding line being essentially aligned with the upper free edge of the front wall, said package being sealed the inner face to the outer face in the overlapping portion between the flap and the front wall by means of a longitudinal welding seam, and welded inner face to outer face along two side edge portions, and outer face to inner face in the adjacent areas of the overlapping portion for obtaining a closed package enclosing a packed product, said packing material providing a peel seal when welded inner face to outer face, and a strong seal (fusion seal) when welded inner face to inner face,
said wipe being **characterized in that** seen relative to the package the packing material comprises an inner layer comprising polyethylene (PE), an outer layer comprising a copolymer of polyethylene and polypropylene and a subjacent intermediate layer comprising polypropylene (PP).

17. The wipe of claim 16, wherein the inner layer is a copolymer of PE and PP differing from the copolymer in the outer layer.
